# EUROPEAN PATENT APPLICATION

(11) **EP 4 741 020 A1**
(43) Date of publication of application: **13.05.2026**
(21) Application number: 24836408.5
(22) Date of filing: 28.06.2024
(51) Int. Cl.: A61Q 19/00, A61Q 17/00, A61K 31/728, A61K 33/38, A61K 8/18, A61K 8/27, A61K 8/29, A61K 8/67, A61P 17/10, A61P 17/16

(54) **NANO PROTECTOR FORMULATION BASED ON HYALURONIC ACID AND AMINO ACIDS FOR SKIN CARE AND REPAIR**

(30) Priority: 05.07.2023 MX 2023008058
(71) Applicant: Bionano Research, S.A. De C.V., Ciudad de Mexico, 10200 (MX)
(72) Inventor: VAZQUEZ HERNANDEZ, Victor Elías, CIUDAD DE MEXICO, 10200 (MX); VAZQUEZ HERNANDEZ, Patricia Guadalupe, CIUDAD DE MEXICO, 10200 (MX)
(74) Representative: Temiño Ceniceros, Ignacio
(86) International application number: PCT/MX2024/050036
(87) International publication number: WO 2025/009962

(57) **Abstract**

The present invention relates to a new formulation based on hyaluronic acid and other amino acids that hydrate, repair, brighten, nourish , and protect the skin and defend it topically against all pathogens, but which has a completely different mechanism of action from those disclosed in the prior art. The use, preparation method and dosage forms of the formulation provide a set of cosmetic and medical products that include zinc, TiO2, rosewater, tannins and vitamins to achieve the technical effect stated in the application.

## Description

### FIELD OF THE INVENTION

The present invention relates to a new formulation based on hyaluronic acid and other amino acids that hydrate, repair, brighten, nourish, and protect the skin and defend it topically against all pathogens, but which has a completely different mechanism of action from those disclosed in the prior art. Therefore, is considered to be a biotechnological invention.

### BACKGROUND

Various applications of hyaluronic acid in cosmetic inventions, particularly its inclusion in formulations with different compounds that help or allow its absorption into the skin, have been disclosed in the state of the art, so comparisons of the effects achieved by the different formulations could begin to be made. In this case, it can be found in the state of the art at least one formulation that delivers basic organic molecules and amino acids to the skin, however, in a completely separate manner, each one of them serves some technical effect such as reducing hyperpigmentation, hydrating, stimulating collagen production and eliminating bacteria, fungi and viruses that may affect the skin, some others allow repairing skin tissue, generating a polarising effect and protecting against radiation; such as those set out below.

Russian patent RU2138245 (C1) entitled "Cosmetic composition for the protection of skin and/or hair from ultraviolet rays, method for the cosmetic protection of skin and/or hair from ultraviolet radiations, agent for reducing the composition containing titanium dioxide pigments, method for reducing the composition containing titanium dioxide-based pigments"; this invention relates to a substantially anhydrous liquid concentrate which comprises dissolved micronutrients in an amount between 0.3% by weight and 5% by weight of metal ions, chosen from zinc, manganese, selenium, iron, copper, cobalt, molybdenum and magnesium and mixtures thereof, 0.3% by weight of carboxylic acid compound which is soluble in glycol, and at least 40% by weight of a glycol compound, wherein the glycol compound is a compound comprising an α,ω-ethyldiol group, optionally comprising ethyl ether groups.

The main claims of the invention include a cosmetic composition for protecting skin and/or hair from ultraviolet rays by means of topical application, characterised in that it comprises in a cosmetically acceptable carrier at least one acyl glutamate and at least one amphiphilic ionic lipid selected from acylamino acids.

Argentinian patent AR 069973**,** entitled "Gel technology suitable for use in cosmetic compositions", discloses a gel system comprising a fractal network of nanoparticles and macroscopic particles. The gel system is capable of forming an effective "optical gel" for blurring lines and wrinkles as a result of the size domain differences between fractal particles and macroscopic particles. Cosmetic compositions comprising such gels and methods for use thereof are also disclosed. Claim 2: The cosmetic composition of claim 1, wherein the nanoparticles are inorganic nanoparticles having a particle size ranging from about 50 to 900 nanometres and a refractive index ranging from about 1.38 to about 2. Claim 3: The cosmetic composition of claim 2, wherein the inorganic nanoparticles are silica, alumina, titania, zirconia, zinc oxide, indium-doped tin oxide, cerium, or mixtures thereof and wherein the macroscopic particles are selected from the group consisting of silicone elastomers; silicone crosspolymers; hydrocarbon elastomers; natural and synthetic rubbers; polymeric spheres, and compatible combinations thereof. Claim 4: The composition of claim 3, wherein the polymeric spheres are selected from the group consisting of fluoropolymers, polyacrylates, nylon, polyesters, cellulose capsules, polyurethanes, polyacrylic esters, polyethers, polyamides, polyesteramides, polyurethanes, and mixtures thereof. Claim 14: The cosmetic composition of claim 3, wherein the gel is an aqueous dispersion of first nanoparticles and second nanoparticles having a specific pH, and the first and second nanoparticles have oppositely charged zeta potentials at said pH. Claim 16: The cosmetic composition of claim 3, wherein the cosmetic composition is substantially anhydrous and the gel further comprises a non-aqueous solvent. Claim 21: A substantially anhydrous cosmetic composition comprising a gel comprising (a) a fractal network of nanoparticles; (b) translucent macroscopic particles and (c) a non-aqueous solvent, and the refractive index of the nanoparticles does not match the refractive index of the macroscopic particles. Claim 32: A method for optically blurring the appearance of skin imperfections selected from the group consisting of wrinkles, fine lines, and pores comprising the step of applying to the skin an amount of a skin care or make-up composition effective to optically blur the appearance of said skin imperfection, and the composition comprises a gel system comprising (a) a fractal network of nanoparticles; and (b) translucent macroscopic particles.

Spanish patent ES2207919 entitled "Artificial tanning compositions comprising iron oxide nanopigments and uses" relates to cosmetic and/or dermatological compositions intended for artificially tanning the skin to such extent as to resemble a natural tan, comprising, in a support in the form of a water-in-oil emulsion, more than 2% by weight of at least one iron oxide nanopigment, the mean dimension of the primary particles of which is less than 100 nm. The invention also relates to the use of a water-in-oil emulsion containing at least one iron oxide nanopigment in, or for the preparation of, cosmetic and/or dermatological products, for artificially tanning the skin to such extent as to resemble a natural tan. The invention also relates to a method for artificially tanning the skin to such extent as to resemble a natural tan using said compositions.

Canadian patent CA1140469 (A) entitled "Hyaluronate-based compositions and cosmetic formulations containing same" discloses a water-based, viscoelastic composition for use in cosmetic formulations which comprises:
(a) a mixture of sodium hyaluronate fractions having different molecular weights,
(b) protein derived from the natural material from which the hyaluronate is obtained, and
(c) water.

The invention also describes cosmetic formulations comprising about 0.05 - 5.0% of the above composition together with an emollient, a sugar alcohol, a neutral or anionic polysaccharide, a preservative, bacteriostatic and fungistatic substance which does not react with or degrade hyaluronic acid and water.

Finally, publication WO2004/096863 A1 discloses a method for purifying a high molecular weight hyaluronic acid molecule; wherein the method for purifying high molecular weight hyaluronic acid from a natural hyaluronic acid compound containing proteins and hyaluronic acid includes the steps of: hydrolysing the proteins contained in the natural hyaluronic acid compound and then performing a first high-speed centrifugation to separate the hydrolysed proteins from the hyaluronic acid; and performing a second high-speed centrifugation to separate high molecular hyaluronic acid and low molecular hyaluronic acid from the centrifuged hyaluronic acid. According to the present invention, hyaluronic acid and proteins can be separated from one another in a cost-effective manner using high-speed centrifugation with low power consumption. Furthermore, highly purified high molecular weight hyaluronic acid and low molecular weight hyaluronic acid can be obtained separately.

These inventions, including methods, formulations and compositions are different from the invention proposed for registration in this document; as the formulation that delivers basic organic molecules and amino acids to the skin reduces hyperpigmentation, hydrates, stimulates collagen production and eliminates bacteria, fungi and viruses that may affect the skin, repairs skin tissue, generates a polarising effect (as a result of nanotechnology) and protects against radiation.

Moreover, the bacterium responsible for the effects of acne is known as *Cutibacterium acnes* (measuring 500 nm in diameter and up to 5 micrometres in length) which is a Gram-positive bacterium that thrives only in oily environments.

Furthermore, *Malassezia* is a genus of fungi. It is normally found in the skin of animals, including humans, and it is the cause of dandruff and an infectious, non-contagious skin disease called pityriasis versicolor.

Scars form when the outermost layer of the skin, the epidermis, is damaged and the deeper layers of the skin are also affected. The skin is not able to regenerate as effectively as usual and this wound will be replaced by a granular tissue that is composed of collagen fibres.

Therefore, this formulation delivers basic organic molecules and amino acids to the skin, reduces hyperpigmentation, hydrates, stimulates collagen production and eliminates bacteria, fungi and viruses that may affect the skin, repairs skin tissue, generates a polarising effect (as a result of nanotechnology) and protects against radiation.

Among the technical effects included in this invention, it has been identified that the invention is developed based on the physical principles of some nanoparticles and the stability of organic substances. In addition to protecting the skin against UV rays, it provides basic organic molecules and amino acids for the proper functioning of the skin and a rejuvenated appearance, reduces hyperpigmentation (reduces spots), hydrates, stimulates collagen production for tissue repair where it is applied, and is mainly responsible for eliminating bacteria, fungi and viruses that may affect the skin, thus creating a shield against new external agents that want to enter and a cage where those that are already causing problems are eliminated. All this in an absolute synergy that allows it to eliminate these microorganisms while repairing the tissue without damaging cells and their DNA, generating at the same time a polarising effect (as a result of nanotechnology) which prevents a greater amount of radiation-induced heat from entering. All the elements used herein are legal for international use and are not subject to any restrictions (in the manner in which the inventor works with them).

### DESCRIPTION

The characteristic details of this new nano protector formulation based on hyaluronic acid and amino acids for skin care and repair are clearly shown in the following description and in the accompanying figures where the same reference signs are used to indicate the parts and figures shown.

The components of the same inventive concept can be separated based on the above figures and in order to provide clarity to the description it will be separated as follows:
a) The formulation
b) The method
c) The use
d) The dosage forms

**THE FORMULATION (I).** The formulation obtained with the above method comprises, as can be seen, two homogeneous mixtures which, when taking one unit of the formulation, each compound is present in the mixture per 100 ml. The following compounds to obtain a formulation are present in the following ranges and proportions:
i. At least two aqueous extracts; the former being present in the mixture in a concentration range comprised between 2% and 4% and the second in a range comprised between 8% and 12%;
ii. Vitamins: at least in three types of different concentrations: the first being vitamin C (ascorbic acid) in a range comprised between 8% and 12%; the second being vitamin E (tocopherol acetate) in a range comprised between 3% and 7%; the third being vitamin B5 (D-panthenol with allantoin) in a range comprised between 2% and 6%;
iii. Hydrolysed hyaluronic acid in a range comprised between 2% and 3%;
iv. Nanoparticles of two types in different concentrations; the first being dry sphere silver (Ag) nanoparticles having a size comprised in a range of between 70 nm and 100 nm, present in the mixture in a range comprised between 0.5% and 1.5%; the second being dry sphere copper oxide (CuO) nanoparticles having a size comprised in a range of between 30 nm and 60 nm, present in the mixture in a range comprised between 0.1% and 1%;
v. Pycnogenol, present in the mixture in a range comprised between 2% and 6%;
vi. Tannins (preferably in powder form) present in the mixture in a range comprised between 2% and 4%; and
vii. A generic base or vehicle containing zinc and TiO₂ physical filters only;

This formulation allows hydrating, repairing, brightening, nourishing, protecting the skin and defending it topically against all pathogens, as will be demonstrated below.

**THE METHOD.** The method for generating formulation (I) comprises the following steps:
a. **PREPARING THE BASE: FORMULATION (mA).** The vehicle consisting of a generic gel with zinc oxide and TiO₂ is poured into a sterilised (preferably glass) container using only physical filters and left to stand for at least ten minutes at a temperature comprised between 17° and 25°C; rosewater is added stirring vigorously at a constant speed of preferably 50 rpm for at least two minutes; tannins, preferably in powder form, are then added increasing the stirring speed to a range between 110 and 130 rpm until obtaining a homogeneous mixture without agglutination and left to stand for at least 15 minutes; (this preparation is **mA)**
b. **PREPARING mA ADDED WITH VITAMINS. FORMULATION (mB).** Once the standing time of the preceding step has elapsed, the mixture **mA** is heated until its temperature is raised to at least 55°C stirring constantly throughout the entire period in which the temperature rises at a speed of 50 rpm until the homogeneity thereof is achieved every time that each vitamin is added and, without stopping the stirring, vitamin B5 is first added, then vitamin E and finally vitamin C, continuing the stirring for at least three more minutes; this mixture is left to stand for the time necessary for its temperature to drop to at least 35°C (this preparation is **mB);**
c. **PREPARING HYALURONIC ACID. FORMULATION (mC)** Hyaluronic acid is poured into a container which is different from that used in the preceding steps in controlled temperature conditions, ensuring that it is contained between 20°C and 30°C, pycnogenol and aqueous honey extract are added at a constant speed of 100 rpm; stirring is maintained for at least four minutes and left to stand for at least five more minutes; (this preparation is **mC);**
d. **OBTAINING HOMOGENEOUS MIXING BETWEEN mB and mC. FORMULATION (mD).** When the temperature of the mixture mB obtained has dropped to 35°C, mB is mixed with mC and the temperature is raised again until reaching a range between 35°C and 45°C stirring constantly at 130 rpm for at least five minutes until achieving a homogeneous mixture again and the temperature is lowered to 25°C, leaving to stand in a photosensitive bottle (in this case, a dark bottle) to prevent oxidation of the formulation and the controlled temperature must be maintained for at least ten more minutes; (this preparation is **mD);**
e. **ADDING NANOPARTICLES.** Once the standing time of the preparation mixture of the preceding step has elapsed, it is stirred in a range comprised between 130 and 160 rpm for pouring in silver (Ag) nanoparticles until no apparent agglutination is observed and left to stand for at least seven minutes; it is then stirred again in the same range and copper oxide (CuO) nanoparticles are added and it is left to stand again for at least ten minutes; finally it is packaged in a photosensitive container (preferably an amber container) and, in order to maintain the optimal conditions of the mixture, it is kept in a cool dry place at a temperature comprised between 25°C and 33°C;

**USES.** Formulation (I) prepared with the method described above has the following uses:
a. Adjuvant in the treatment of acne, dermatitis, and other diseases caused by viruses, bacteria and fungi that are infecting the skin;
b. Skin healing;
c. Anti-inflammatory in skin cancer prevention;
d. Reduction of spots (hyperpigmentation); and
e. Elimination of premature fine lines.

Now a formulation with effects of hydrating, repairing, brightening, nourishing, protecting the skin and defending it against all pathogens has been obtained; now in order to support the above uses and based on the results of scientific observations developed with the application of the formulation obtained for the declared uses, all the foregoing will be illustrated by the following examples which are intended to be fully illustrative and not limiting.

### Example 1. Treatment against skin pathogens and acne.

Skin problems such as acne, dermatitis, redness, etc. of the skin, is caused by the presence of bacteria, viruses, fungi and other vectors of these diseases.

Bacterial skin infections often start as small red bumps that slowly increase in size. Some bacterial infections are mild and easily treated with topical antibiotics, but other infections require an oral antibiotic. Different types of bacterial skin infections include: cellulitis, impetigo, boils, leprosy, among others. Viral infections include: shingles (herpes zoster), chickenpox, molluscum contagiosum, warts, measles and hand, feet and mouth diseases. Fungal infections include those mainly found in moist areas of the body, such as feet or armpits. Some infections are not contagious, and these infections such as athlete's foot, fungal infection, ringworm, nail fungus, oral thrush and nappy rash are usually not life-threatening; finally parasitic infections which are caused by a parasite. These infections can spread beyond the skin into the bloodstream and organs. A parasitic infection, such as head lice, bedbugs, scabies and cutaneous larva migrans, is not life-threatening, but can be annoying.

In this example, parasitic infections are not taken into consideration as the formulation is for topical applications and not invasive with respect to the bloodstream.

In one experiment, symptoms of skin infection are analysed, said symptoms being: the presence of irritation, rash; vector integration due to a cut or scratch; the presence of infection such as poxvirus, HPV or herpes; and finally humidity and temperature conditions.

In four groups formed as control group A and groups B, C and D with exposure to different concentrations of formulation (I) in a set of 30 study subjects per group, the following observations of Table I were obtained.

The number of subjects exhibiting contamination was counted by the type of vector and the type of damage observed and grouped to perform application at different concentrations.

Objective. Initial identification of the conditions of each group and the number of individuals having the vector and the type of damage caused. For the application of the formulation, the time was kept constant in all applications and over the same period of time, constant climatic conditions (32°C) and 60% relative humidity in the environment. The following initial conditions are thus established.

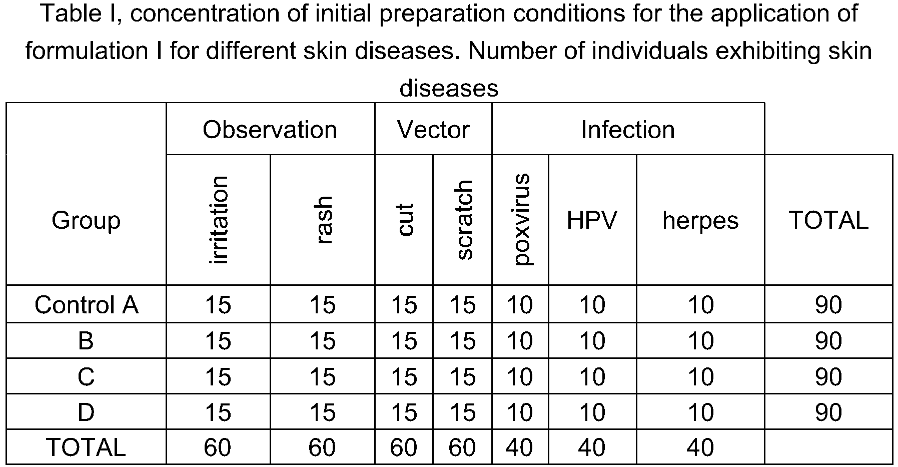

Concentration of formulation (I) to be applied by groups:

| | |
|---|---|
| Group A (control) | 0% |
| Group B | 10% |
| Group C | 25% |
| Group D | 43% |

Observation was carried out in three observation periods of 10 days each; individuals exhibiting any of the following three levels for each of the indicators studied were selected.
1 minor
2 moderate
3 serious

Thus, the initial observation conditions were those shown in Table 2.

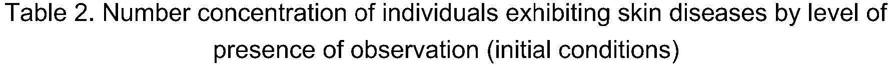

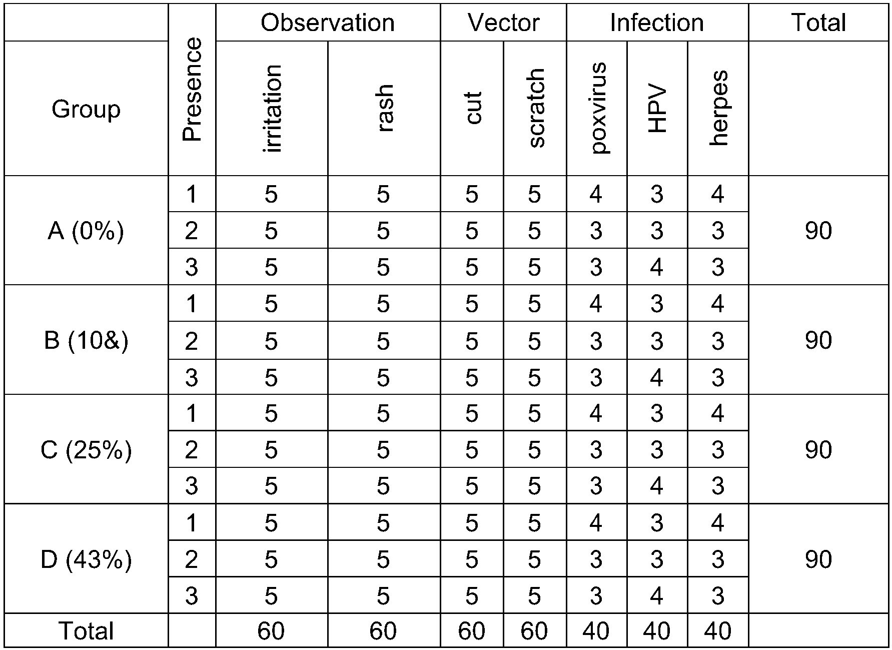

When the different groups were exposed to concentrations, the following changes were observed in each of the groups exposed to the formulation, these results are shown in Table 3.

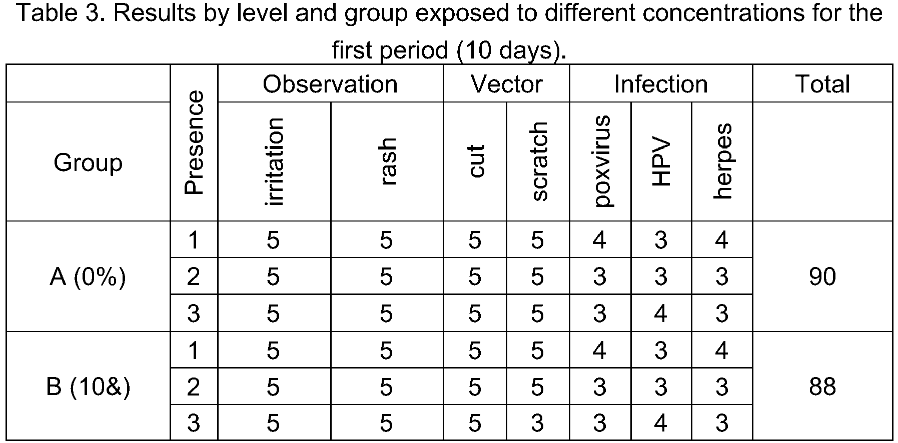

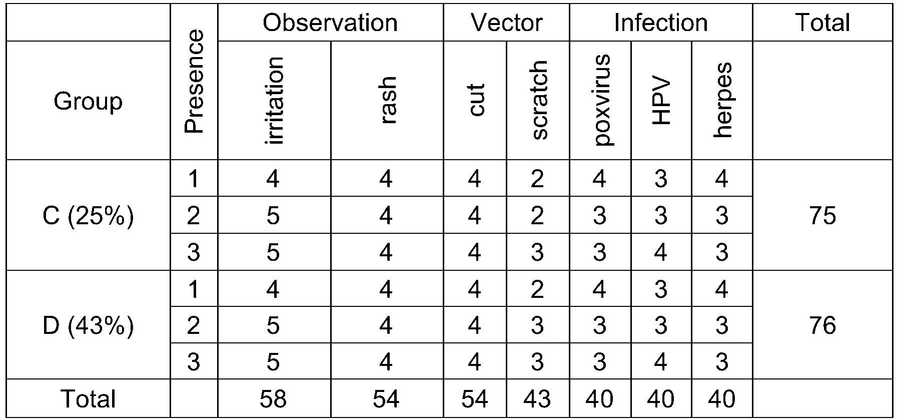

With the concentration, a decrease in the number of individuals due to the action of the formulation can be seen, these individuals being considered without the observation of the initial condition.+

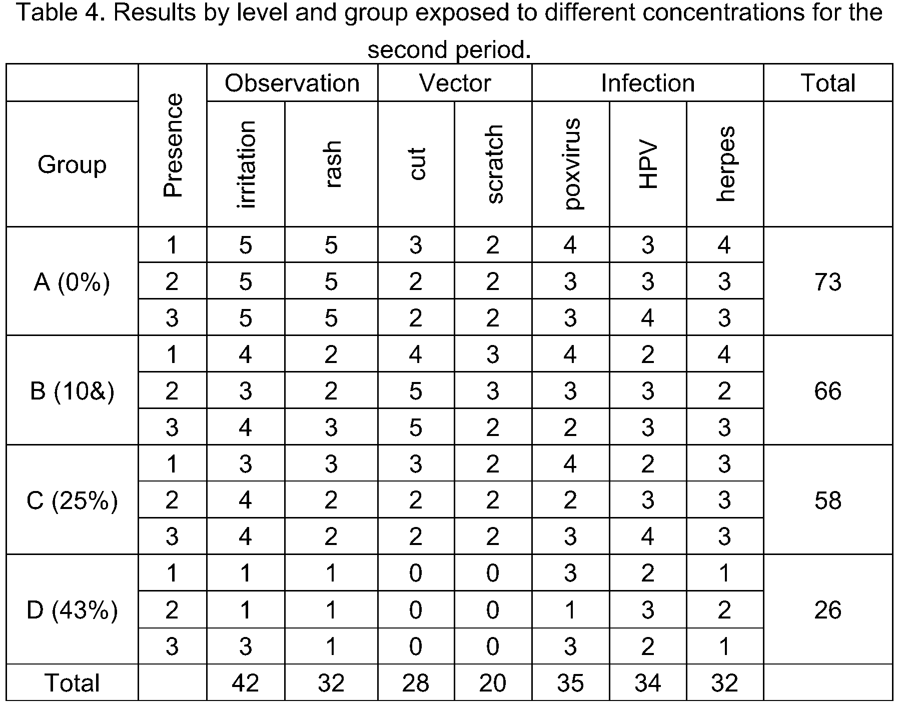

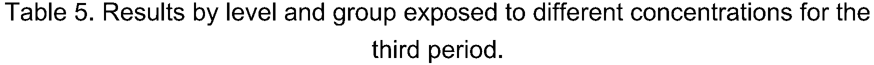

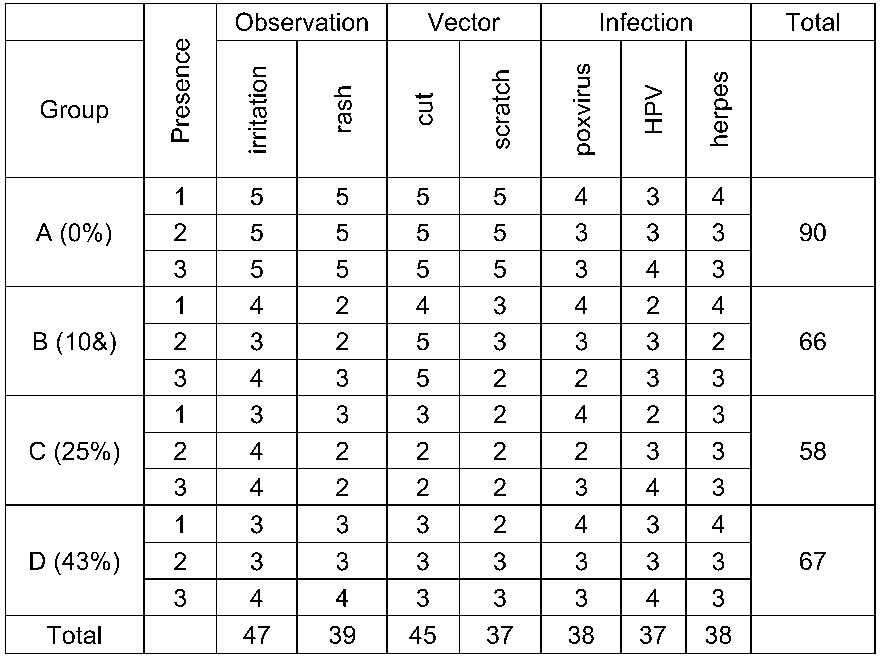

### Conclusions:

The highest percentage of concentrations of formulation I has shown significant progress for samples C and D, particularly due to the timing of the application of the formulation, although growth is not exponential, it does not have a linear behaviour either, because the difference between group B and these two is not constant and it is noted that as individuals in the control group slowly improve without the formulation, in contrast to group D, i.e., the higher the concentration and the longer the exposure time, the better the patient becomes, it is clear that in the case of skin diseases, its action is significant compared to those which are infectious diseases.

That being said, it may be noted that cutaneous application is favourable for improvement, so it is necessary to observe the resistance thereof to UV rays. Example 2. Resistance of formulation (I) to UV rays.

Melatonin is produced only in the brain, although it has also been found to arise from certain skin cells. In fact, recent studies corroborate that melatonin is capable of suppressing UV ray-induced damage on skin cells and functions as an antioxidant, which means that it protects cellular DNA from free radical damage. Taking into account that sun damage is the main cause of the signs of ageing, such as wrinkles, fine lines and dark spots, all indications are that melatonin combats these signs of ageing.

Taking melatonin before sun exposure reduces the risk of sunburn and its application as a 0.5% cream is the most potent concentrate capable of eliminating erythema or redness caused by sunburn (ultraviolet light). People prone to atopic dermatitis can mitigate its effects by taking and applying melatonin.

In a preferred preparation of formulation (I) as follows:
The formulation has the following ranges and proportions:
a. At least 30% of the formulation
b. At least 20% of 0.5% melatonin
c. An excipient.

The second aqueous extract present in the formulation is responsible for saturating melatonin and acts synergistically with the molecule; melatonin is poorly soluble in water or other aqueous biological fluids, however, to achieve saturation, it is stirred vigorously until achieving a homogeneous mixture, but it can potentially be administered in an undissolved state, such as in currently available melatonin products; the drug remains undissolved and, therefore, must be swallowed in order to be absorbed in substantial amount.

This formulation releases melatonin in an undissolved state and the absorption of the drug is limited by how quickly the drug dissolves at the site of local administration. Therefore, it is necessary to place it on the skin, but at the time of placing same, there is a need for a fixing agent such as hyaluronic acid which retains the aqueous mixture with melatonin on the surface, leaving it trapped between the skin and the acid which in the formulation, zinc and TiO₂ which soften and retain more UV rays, allowing melatonin to remain in contact with the skin and the pores to remain open when sweating, so it successfully penetrates the dermis, providing protection against the action of UV rays. In accordance with the weather dictionary, human beings are characterised into six skin types presented in Table 6.

**Table 6. Skin type weather dictionary)**

| Skin type | Skin type | Skin type |
|---|---|---|
| I | Alabaster white skin with many freckles, blonde | Always burns easily, burn may be intense. Never tans |
| II | White skin blue eyes | Always burns easily, burn may be intense. May tan, but minimally. |
| III | White skin with minimal brown tone; Caucasians | May burn, burn is moderate. May tan gradually |
| IV | More or less intense brown skin tone. Mediterraneans | May burn, but always minimally. Always tans. |
| V | Dark brown skin tone, Asians, Blacks with less intense tone, Middle Eastern people, South Americans. | Rarely burns, tans intensely. |
| VI | Blacks with intense tone, | Never burns, tans intensely. |

Six groups of 10 people, one per skin type, were selected and exposed directly to the proposed formulation and two other commercial formulations, observing the results of Table 7, where the control group (sample) starts with a percentage of melatonin and was exposed for a time of 20 min considered as the average between a mild injury and a severe injury; measurement of the initial percentage thereof and the final percentage together with the average percentage of UV ray penetration into the skin were performed in order not to expose the study subjects, the percentage change in the main levels measured were as shown in the following table.

**Table 7. Melatonin variation in 10 study subjects exposed for 20 minutes with different products.**

| Skin type / formulation | Sample | Formulation (I) | | Commercial I | | Commercial II | |
|---|---|---|---|---|---|---|---|
| | | initial | final | initial | final | initial | final |
| I | T. 20 | M. 22% | M. 18% | M. 22% | M. 10% | M. 22% | M. 11% |
| | | UV 27% | UV 11% | UV 27% | UV 40% | UV 27% | UV 37% |
| II | T. 15 | M. 20% | M. 20% | M. 20% | M. 19% | M. 20% | M. 17% |
| | | UV 30% | UV 13% | UV 30% | UV 10% | UV 30% | UV 9% |
| III | T. 15 | M. 16% | M. 15% | M. 16% | M. 14% | M. 16% | M. 15% |
| | | UV 28% | UV 9% | UV 28% | UV 29% | UV 28% | UV 19% |
| IV | T. 10 | M. 10% | M. 9% | M. 10% | M. 8% | M. 10% | M. 12% |
| | | UV 17% | UV 10% | UV 17% | UV 17% | UV 17% | UV 15% |
| V | T. 10 | M. 8% | M. 5% | M. 8% | M. 7% | M. 8% | M. 7% |
| | | UV 10% | UV 4% | UV 10% | UV 9% | UV 10% | UV 7% |
| VI | T.5 | M. 5% | M. 3% | M. 5% | M. 5% | M. 5% | M. 4% |
| | | UV 10% | UV 3% | UV 10% | UV 9% | UV 10% | UV 8% |

Wherein:
- T. minimum time it takes for the skin to burn without photoprotection.
- M. Melatonin level measured in relation to skin type.
- UV percentage of decrease in UV rays on skin.

### Conclusions:

Melatonin present in the skin increases with the placement of the formulations on the skin of the study subjects and therefore increases from its conventional value, however once it has been measured at the time of starting the experiment, the time is counted and measured again at the end to obtain the controlled conditions of the experiment.

The formulation maintains the melatonin present in the skin and at the same time allows repelling the percentage of UV rays at the end of the experiment more efficiently than the commercial brands against which it is compared.

That being said, the appropriate uses of the formulations that have been demonstrated in the set of presented examples are complemented by the cosmetic and medical dosage forms of the formulation:

### COSMETIC DOSAGE FORMS

| | |
|---|---|
| a. | Topical cream |
| b. | Gel for delicate skin areas |
| c. | Bath gel |
| d. | Body cream |

### MEDICAL DOSAGE FORMS

| | |
|---|---|
| e. | Capsule |
| f. | Lyophilisate |
| g. | Tablet |
| h. | Gel |

The formulation (I) could achieve a HYDRATING effect when mixed with at least one lipid from the following group: ceramides (50-60%), cholesterol and polyunsaturated fatty acids; being present in one unit (100 ml) as follows:
a) The formulation in a preferred range comprised between 1% and 45% present in the mixture;
b) At least one lipid in a preferred range comprised between 99% and 55% present in the mixture; and
c) An excipient.

The formulation (I) could achieve a REPAIRING effect when mixed with collagen; being present in one unit (100 ml) as follows:
a) The formulation in a preferred range comprised between 5% and 55% present in the mixture;
b) Collagen in a preferred range comprised between 95% and 45% present in the mixture; and
c) An excipient.

The formulation (I) could achieve a DEPIGMENTING effect when mixed with at least one acid from the following group: phytic acid, lipoic acid, ellagic acid, lactic acid, salicylic acid, azelaic acid, together with plant extracts derived from bearberry, liquorice or mulberry; being present in one unit (100 ml) as follows:
a) The formulation in a preferred range comprised between 1% and 45% present in the mixture;
b) At least one acid in a preferred range comprised between 99% and 55% present in the mixture; and
c) An excipient.

The formulation (I) could achieve a NUTRITIVE effect when mixed with at least one vitamin from the following group: vitamin A, B, C, D, E, K; being present in one unit (100 ml) as follows:
a) The formulation in a preferred range comprised between 1% and 45% present in the mixture;
b) At least one vitamin in a preferred range comprised between 99% and 55% present in the mixture; and
c) An excipient.

The formulation (I) could achieve a PROTECTIVE effect when mixed with at least one compound from any of the group: methyl cinnamate, avobenzone, octisalate; salicylate; being present in one unit (100 ml) as follows:
a) The formulation in a preferred range comprised between 1% and 45% present in the mixture;
b) At least one vitamin in a preferred range comprised between 99% and 55% present in the mixture; and
c) An excipient.

## Claims

1. A method for preparing a nano protector formulation based on hyaluronic acid and amino acids for skin care and repair, **characterised in that** it comprises the following steps:
a. **PREPARING THE BASE: FORMULATION (mA).** The vehicle consisting of a generic gel with zinc oxide and TiO2 is poured into a sterilised (preferably glass) container using only physical filters and left to stand for at least ten minutes at a temperature comprised between 17° and 25°C; rosewater is added stirring vigorously at a constant speed of preferably 50 rpm for at least two minutes; tannins, preferably in powder form, are then added increasing the stirring speed to a range between 110 and 130 rpm until obtaining a homogeneous mixture without agglutination and left to stand for at least 15 minutes; (this preparation is **mA)**
b. **PREPARING mA ADDED WITH VITAMINS. FORMULATION (mB).** Once the standing time of the preceding step has elapsed, the mixture **mA** is heated until its temperature is raised to at least 55°C stirring constantly throughout the entire period in which the temperature rises at a speed of 50 rpm until the homogeneity thereof is achieved every time that each vitamin is added and, without stopping the stirring, vitamin B5 is first added, then vitamin E and finally vitamin C, continuing the stirring for at least three more minutes; this mixture is left to stand for the time necessary for its temperature to drop to at least 35°C (this preparation is **mB);**
c. **PREPARING HYALURONIC ACID. FORMULATION (mC)** Hyaluronic acid is poured into a container which is different from that used in the preceding steps in controlled temperature conditions, ensuring that it is contained between 20°C and 30°C, pycnogenol and aqueous honey extract are added at a constant speed of 100 rpm; stirring is maintained for at least four minutes and left to stand for at least five more minutes; (this preparation is **mC);**
d. **OBTAINING HOMOGENEOUS MIXING BETWEEN mB and mC. FORMULATION (mD).** When the temperature of the mixture mB obtained has dropped to 35°C, mB is mixed with mC and the temperature is raised again until reaching a range between 35°C and 45°C stirring constantly at 130 rpm for at least five minutes until achieving a homogeneous mixture again and the temperature is lowered to 25°C, leaving to stand in a photosensitive bottle (in this case, a dark bottle) to prevent oxidation of the formulation and the controlled temperature must be maintained for at least ten more minutes; (this preparation is **mD);**
e. **ADDING NANOPARTICLES.** Once the standing time of the preparation mixture of the preceding step has elapsed, it is stirred in a range comprised between 130 and 160 rpm for pouring in silver (Ag) nanoparticles until no apparent agglutination is observed and left to stand for at least seven minutes; it is then stirred again in the same range and copper oxide (CuO) nanoparticles are added and it is left to stand again for at least ten minutes; finally it is packaged in a photosensitive container (preferably an amber container) and, in order to maintain the optimal conditions of the mixture, it is kept in a cool dry place at a temperature comprised between 25°C and 33°C.

2. A formulation obtained with the method of claim 1, **characterised in that** it comprises two homogeneous mixtures which, when taking one unit of the formulation, each compound is present in the mixture per 100 ml:
i. At least two aqueous extracts; the former being present in the mixture in a concentration range comprised between 2% and 4% and the second in a range comprised between 8% and 12%;
ii. Vitamins: at least in three types of different concentrations: the first being vitamin C (ascorbic acid) in a range comprised between 8% and 12%; the second being vitamin E (tocopherol acetate) in a range comprised between 3% and 7%; the third being vitamin B5 (D-panthenol with allantoin) in a range comprised between 2% and 6%;
iii. Hydrolysed hyaluronic acid in a range comprised between 2% and 3%;
iv. Nanoparticles of two types in different concentrations; the first being dry sphere silver (Ag) nanoparticles having a size comprised in a range of between 70 nm and 100 nm, present in the mixture in a range comprised between 0.5% and 1.5%; the second being dry sphere copper oxide (CuO) nanoparticles having a size comprised in a range of between 30 nm and 60 nm, present in the mixture in a range comprised between 0.1% and 1%;
v. Pycnogenol, present in the mixture in a range comprised between 2% and 6%;
vi. Tannins (preferably in powder form) present in the mixture in a range comprised between 2% and 4%; and
vii. A generic base or vehicle containing zinc and TiO₂ physical filters only.

3. USE of the formulation of claim 2, **characterised in that** it has HYDRATING effects when mixed with at least one lipid from the following group: ceramides (50-60%), cholesterol and polyunsaturated fatty acids; being present in one unit (100 ml) as follows:
a) The formulation in a preferred range comprised between 1% and 45% present in the mixture;
b) A lipid in a preferred range comprised between 99% and 55% present in the mixture; and
c) An excipient.

4. USE of the formulation of claim 2, **characterised in that** it has REPAIRING effects when mixed with collagen; being present in one unit (100 ml) as follows:
a) The formulation in a preferred range comprised between 1% and 55% present in the mixture;
b) Collagen in a preferred range comprised between 95% and 45% present in the mixture; and
c) An excipient.

5. USE of the formulation of claim 2, **characterised in that** it has DEPIGMENTING effects when mixed with phytic acid, lipoic acid, ellagic acid, lactic acid, salicylic acid, azelaic acid, together with plant extracts derived from bearberry, liquorice or mulberry; being present in one unit (100 ml) as follows:
a) The formulation in a preferred range comprised between 1% and 45% present in the mixture;
b) An acid in a preferred range comprised between 99% and 55% present in the mixture; and
c) An excipient.

6. USE of the formulation of claim 2, **characterised in that** it has NUTRITIVE effects when mixed with vitamins A, B, C, D, E, K; being present in one unit (100 ml) as follows:
d) The formulation in a preferred range comprised between 1% and 45% present in the mixture;
e) An acid in a preferred range comprised between 99% and 55% present in the mixture; and
f) An excipient.

7. USE of the formulation of claim 2, **characterised in that** it has PROTECTIVE effects when mixed with a compound from any of the group: methyl cinnamate, avobenzone, octisalate; salicylate; being present in one unit (100 ml) as follows:
a) The formulation in a preferred range comprised between 1% and 45% present in the mixture;
b) At least one compound in a preferred range comprised between 99% and 55% present in the mixture; and
c) An excipient.

8. THE formulations of claims 2 to 7, **characterised in that** their dosage form is a TOPICAL CREAM which allows direct application on skin before sun exposure in a time range that ranges between 10 and 50 min.

9. THE formulations of claims 2 to 7, **characterised in that** their dosage form is a GEL which allows direct application on skin after sun exposure in a time range that ranges between 30 and 60 min.

10. THE formulations of claims 2 to 7, **characterised in that** their dosage form is a BODY CREAM which allows direct application on skin after bath or shower, in a time range that ranges between 0.1 and 20 min.

11. THE formulations of claims 2 to 7, **characterised in that** their dosage form is a CAPSULE which allows ingestion in a dose of 100 mg every 24 hours for 3 days prior to the time of sun exposure.

12. THE formulations of claims 2 to 7, **characterised in that** their dosage form is a TABLET which allows ingestion in a dose of 100 mg every 24 hours for 3 days prior to the time of sun exposure.

13. THE formulations of claims 2 to 7, **characterised in that** their dosage form is a LYOPHILISED POWDER which allows reconstitution with double distilled water prior to its application.
